# EUROPÄISCHE PATENTANMELDUNG

(11) **EP 1 431 273 A1**
(43) Veröffentlichungstag der Anmeldung: **23.06.2004**
(21) Anmeldenummer: 03027758.6
(22) Anmeldetag: 03.12.2003
(51) Int. Cl.: C07C 209/64

(54) **Verfahren zur Herstellung eines symmetrischen sekundären Amins**

(30) Priorität: 20.12.2002 DE 10261195
(71) Anmelder: BASF AKTIENGESELLSCHAFT, 67056 Ludwigshafen (DE)
(72) Erfinder: Gerlach, Till, Dr., 67071 Ludwigshafen (DE); Funke, Frank, Dr., 67067 Ludwigshafen (DE); Benisch, Christoph, Dr., 68165 Mannheim (DE); Melder, Johann-Peter, Dr., 67459 Böhl-Iggelheim (DE)

(57) **Zusammenfassung**

Verfahren zur Herstellung eines symmetrischen sekundären Amins durch Umsetzung eines primären Amins in Gegenwart von Wasserstoff und eines Katalysators, wobei man einen Katalysator einsetzt, bei dessen Herstellung eine Fällung von katalytisch aktiven Komponenten auf monoklines, tetragonales oder kubisches Zirkoniumdioxid erfolgte.

## Beschreibung

Die vorliegende Erfindung betrifft ein Verfahren zur Herstellung eines symmetrischen sekundären Amins (R-NH-R; 'symmetrisch' bringt zum Ausdruck, dass die beiden Reste R gleich sind) durch Umsetzung eines primären Amins (R-NH₂) in Gegenwart von Wasserstoff und eines Katalysators.

Verfahren zur Herstellung von symmetrischen sekundären Aminen aus primären Aminen sind an sich bekannt (Dimerisierung des primären Amins in Gegenwart von H₂ unter NH₃-Bildung nach: 2 R-NH₂ + H₂ → R-NH-R + NH₃). Es ist dabei Stand der Technik, ein primäres Amin, das die gewünschten Substituenten bzw. Strukturelemente aufweist, bei den jeweils gewählten Reaktionsbedingungen unter Wasserstoff zu dem gewünschten sekundären Amin umzusetzen. Dabei werden verschiedenste Katalysatoren eingesetzt; die bei der Reaktion angewandten Drücke und Temperaturen variieren stark. Ein Hauptproblem bei der beschriebenen Synthese der symmetrischen sekundären Amine liegt in dem Umsatz bzw. der Selektivität zu dem gewünschten Produkt, der/die häufig nicht die gewünschten Werte erreicht. Oft erweist sich auch die Verwendung teurer edelmetallhaltiger Katalysatoren als notwendig.

DE-A-30 48 832 (Texaco) betrifft ein Verfahren zur Herstellung von Bis-alkylaminen, insbesondere Bis-[(3-dimethylamino)propyl]amin (Bis-DMAPA) aus 3-(Dimethylamino)propionitril (DMAPN) oder 3-(Dimethylamino)propylamin (DMAPA) bzw. Gemischen von DMAPN und DMAPA. In einem Beispiel wird bei hohem Druck (173 bar) an Ni-Cu-Cr₂O₃ bei einem DMAPA-Umsatz von 53 % eine Bis-DMAPA-Selektivität von 80 % erreicht, an Co-Cu-Cr₂O₃ bei einem DMAPA-Umsatz von 49 % eine Bis-DMAPA-Selektivität von 88 %.

Es zeigt sich hier, dass insbesondere die Umsätze bei verbesserungswürdigen Werten liegen. Auch ist aufgrund von Umweltaspekten die Verwendung von chromfreien Katalysatoren bevorzugt.

EP-A-412 613 (Union Carbide) betrifft ein Verfahren zur Herstellung von Aminen durch Kondensation von Amino-Verbindungen, wie Alkylenaminen, in Gegenwart von metallischen Phosphatkatalysatoren.

US 5,166,442, US 5,288,909 und US 5,554,793 (Dow) beschreiben ein Reforming-Verfahren für Alkylenamine in Gegenwart von Gruppe-VB-Metalloxiden, Gruppe-VB-Metallphosphaten, Gruppe-IIA-Metallsilikaten und Wolframoxiden.

US 5,243,078 (Air Products) offenbart ein Herstellverfahren für bestimmte Polyalkylenpolyamine aus linearen Alkylenaminen in Gegenwart von Mordenit-Katalysatoren.

Die ältere, nicht vorpublizierte EP-Anmeldung Nr. 02013584.4 (BASF AG) betrifft ein Verfahren zur Herstellung von bestimmten sekundären Aminen aus primären Aminen in Gegenwart von bestimmten Übergangsmetallkatalysatoren.

Eine parallele deutsche Patentanmeldung (BASF AG) mit gleichem Anmeldetag beschreibt ein Verfahren zur Herstellung eines Amins durch Umsetzung eines primären oder sekundären Alkohols, Aldehyds oder Ketons mit Wasserstoff und einer Stickstoffverbindung, ausgewählt aus der Gruppe Ammoniak, primäre und sekundäre Amine, in Gegenwart eines Katalysators, wobei man einen Katalysator einsetzt, bei dessen Herstellung eine Fällung von katalytisch aktiven Komponenten auf monoklines, tetragonales oder kubisches Zirkoniumdioxid erfolgte.

Eine parallele deutsche Patentanmeldung (BASF AG) mit gleichem Anmeldetag betrifft ein Verfahren zur katalytischen Hydrierung einer aliphatisch ungesättigten Gruppe in einer organischen Verbindung, wobei man einen Katalysator einsetzt, bei dessen Herstellung eine Fällung von katalytisch aktiven Komponenten auf monoklines, tetragonales oder kubisches Zirkoniumdioxid erfolgte.

Nachteilig an den Zirkoniumdioxid-haltigen Heterogenkatalysatoren des Stands der Technik für die Herstellung von symmetrischen sekundären Aminen ist deren erfindungsgemäß erkannte Abnahme der mechanischen Stabilität unter den Reaktionsbedingungen ihres Einsatzes, insbesondere in Gegenwart von Reaktionsmedien, die Wasser enthalten. Eine Folge von mechanisch weniger stabilen Heterogenkatalysatoren ist der häufiger notwendige Katalysatorwechsel im Reaktor und damit eine erniedrigte Raum-Zeit-Ausbeute.

Der vorliegenden Erfindung lag demgemäß die Aufgabe zugrunde, den Nachteilen des Stands der Technik abzuhelfen und ein verbessertes Verfahren zur Herstellung von symmetrischen sekundären Aminen bereitzustellen, wobei ein Katalysator mit verbesserten mechanischen Eigenschaften unter den Reaktionsbedingungen seines Einsatzes verwendet wird und damit die Wirtschaftlichkeit bisheriger Verfahren, insbesondere solcher, in denen Zirkoniumdioxid-haltige Katalysatoren zum Einsatz kommen, zu verbessern.

Erfindungsgemäß wurde erkannt, dass ein Grund für die verbesserungswürdige mechanische Stabilität, d.h. die mechanische Erweichung, der bekannten Zirkoniumdioxid-haltigen Katalysatoren unter Reaktionsbedingungen die Tatsache ist, dass das Zirkoniumdioxid, z.B. bei Anwendung der (Misch)Fäll-Technik, zunächst ganz oder teilweise amorph vorliegt und unter den Bedingungen der mit diesen Katalysatoren katalysierten chemischen Reaktion ganz oder teilweise eine Kristallisation, also Umwandlung der Modifikation, zu tetragonalem, monoklinem oder kubischem Zirkoniumdioxid stattfindet. Bei den Dimerisierungsreaktionen von primären Aminen zu symmetrischen sekundären Aminen werden als Reaktionsbedingungen zumeist eine erhöhte Temperatur (z.B. 50 bis 250 °C) und erhöhter Druck (z.B. 5 bis 350 bar bei Gas/Flüssigphasenreaktionen und Gasphasenrektionen) angewendet.

Demgemäß wurde gefunden, dass durch die Verwendung von Zirkoniumdioxid, das eine unter den Reaktionsbedingungen der Dimerisierung des primären Amins thermodynamisch stabile oder zumindest metastabile Modifikation aufweist, wie monoklines, tetragonales oder kubisches Zirkoniumdioxid, bei der Herstellung eines Zirkoniumdioxid-haltigen Katalysators die mechanische Stabilität der resultierenden Katalysatoren unter den Reaktionsbedingungen, insbesondere in Gegenwart von entsprechenden Reaktionsmedien, die Wasser enthalten, wesentlich erhöht wird.

Gegenstand der Erfindung ist demnach ein Verfahren zur Herstellung eines symmetrischen sekundären Amins durch Umsetzung eines primären Amins in Gegenwart von Wasserstoff und eines Katalysators, welches dadurch gekennzeichnet ist, dass man einen Katalysator einsetzt, bei dessen Herstellung eine Fällung von katalytisch aktiven Komponenten auf monoklines, tetragonales oder kubisches Zirkoniumdioxid erfolgte.

Bei den aufgefällten katalytisch aktiven Komponenten handelt es sich insbesondere um Metallsalze, wobei das Metall aus den Gruppen 8 bis 11 (entsprechend den Nebengruppen VIII und IB) des Periodensystems der Elemente, ganz besonders aus der Gruppe Fe, Co, Ni, Ru, Rh, Pd, Pt und Cu, ausgewählt ist.
Besonders bevorzugt ist das Metall aus der Gruppe Cu, Ni und Co ausgewählt.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

Die katalytisch aktive Masse kann nach Mahlung als Pulver oder als Splitt in das Reaktionsgefäß eingebracht oder bevorzugt, nach Mahlung, Vermischung mit Formhilfsmitteln, Formung und Temperung, als Katalysatorformkörper ― beispielsweise als Tabletten, Kugeln, Ringe, Extrudate (z. B. Stränge) ― in den Reaktor eingebracht werden.

Die Konzentrationsangaben (in Gew.-%) der Komponenten des Katalysators beziehen sich jeweils ― falls nicht anders angegeben ― auf die katalytisch aktive Masse des hergestellten Katalysators vor der Behandlung mit Wasserstoff.

Die katalytisch aktive Masse des Katalysators ist als die Summe der Massen der katalytisch aktiven Bestandteile definiert und enthält, vor der Behandlung mit Wasserstoff, im wesentlichen die katalytisch aktiven Bestandteile monoklines, tetragonales oder kubisches Zirkoniumdioxid (oder Mischungen dieser Modifikationen) und Metallsalze als weitere katalytisch aktive Komponenten.

Die Summe der o.g. katalytisch aktiven Bestandteile, berechnet in oxidischer Form, z.B. als ZrO₂, CuO, NiO und CoO, in der katalytisch aktive Masse vor der Behandlung mit Wasserstoff beträgt im allgemeinen 70 bis 100 Gew.-%, bevorzugt 80 bis 100 Gew.-%, besonders bevorzugt 90 bis 100 Gew.-%, insbesondere 95 bis 100 Gew.-%, ganz besonders bevorzugt >98 bis 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen IA bis VIA und IB bis VIIB und VIII des Periodensystems, enthalten.

Beispiele für solche Elemente bzw. deren Verbindungen sind:
Übergangsmetalle, wie Mn bzw. Manganoxide, Re bzw. Rheniumoxide, Cr bzw. Chromoxide, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat, Zink bzw. Zinkoxide, Silber bzw. Silberoxide, Lanthanide, wie Ce bzw. CeO₂ oder Pr bzw. Pr₂O₃, Alkalimetalloxide, wie Na₂O, Alkalimetallcarbonate, wie Na₂CO₃ und K₂CO₃, Erdalkalimetalloxide, wie SrO, Erdalkalimetallcarbonate, wie MgCO₃, CaCO₃, BaCO₃, Phosphorsäureanhydride und Boroxid (B₂O₃).

Bevorzugte im erfindungsgemäßen Verfahren verwendete Katalysatoren enthalten in ihrer katalytisch aktiven Masse vor der Behandlung mit Wasserstoff
20 bis 85 Gew.-%, bevorzugt 20 bis 65 Gew.-%, besonders bevorzugt 22 bis 40 Gew.-%, monoklines, tetragonales oder kubisches Zirkoniumdioxid (ZrO₂) (oder Mischungen dieser Modifikationen),
1 bis 30 Gew.-%, besonders bevorzugt 2 bis 25 Gew.-%, sauerstoffhaltige Verbindungen des Kupfers, berechnet als CuO, und
14 bis 70 Gew.-%, bevorzugt 15 bis 50 Gew.-%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist,

Besonders bevorzugte Katalysatoren enthalten zusätzlich in ihrer katalytisch aktiven Masse vor der Behandlung mit Wasserstoff
15 bis 50 Gew.-%, besonders bevorzugt 21 bis 45 Gew.-%, sauerstoffhaltige Verbindungen des Kobalts, berechnet als CoO.

Die sauerstoffhaltigen Verbindungen des Kupfers, Nickels und gegebenenfalls Kobalts, jeweils berechnet als CuO, NiO und CoO, der bevorzugten Katalysatoren sind im allgemeinen insgesamt in Mengen von 15 bis 80 Gew.-%, bevorzugt 35 bis 80 Gew.-%, besonders bevorzugt 60 bis 78 Gew.-%, in der katalytisch aktiven Masse (vor der Behandlung mit Wasserstoff) enthalten, wobei besonders bevorzugt das Molverhältnis von Nickel zu Kupfer größer 1 ist.

Die erfindungsgemäß eingesetzten Katalysatoren lassen sich wie folgt herstellen.

Unter Auffällung wird eine Katalysatorherstellung verstanden, bei der ein schwer lösliches Trägermaterial in einer Flüssigkeit, meist Wasser, suspendiert wird, die Dotierungskomponenten, als leicht lösliche Verbindungen eingesetzt, in einer Flüssigkeit, meist Wasser, gelöst und dann durch Zugabe eines Fällungsmittels auf den suspendierten Träger ausgefällt werden. (Vergl. z.B. A. B. Stiles, Catalyst Manufacture, Marcel Dekker, Inc., 1983, S. 15).

Gemäß der vorliegenden Erfindung wird zur Herstellung des Katalysators als Trägermaterial Zirkoniumdioxid in einer thermodynamisch stabilen oder metastabilen Modifikation, d.h. in der monoklinen, tetragonalen oder kubischen Modifikation, eingesetzt.

Die grundlegenden Eigenschaften von Zirkoniumdioxid sind in K. Dyrek, A. Adamski, Z. Sojka, Ceramic Interfaces 2, University Press, Cambridge, 2001, S. 241 - 259
zusammengefasst, so etwa die existierenden Modifikationen monoklin, tetragonal und kubisch und deren Herstellung.

Die Zirkoniumdioxid-Kristallstruktur kann, insbesondere bei der tetragonalen Modifikation, durch Zusätze von einem oder mehreren Metalloxiden der Nebengruppe IIIB oder der Hauptgruppe IIA des Periodensystems, insbesondere Yttriumoxid, Calciumoxid, Lanthanoxid, Magnesiumoxid oder Scandiumoxid, weiter stabilisiert sein.

Diese Stabilisierung bewirkt z.B. ganz oder teilweise eine Hemmung der Umwandlung der tetragonalen Modifikation in die thermodynamisch stabilste monokline Modifikation.

Zur Herstellung des Katalysators wird das Zirkoniumdioxid in einem Lösungsmittel, z.B. in Wasser, suspendiert. Dann werden die z.B. in Wasser gelösten Metallsalze zugegeben und anschließend durch Zugabe von z.B. Lauge in Form von im verwendeten Lösungsmittel, z.B. Wasser, schwer- oder unlöslichen, basischen Salzen gefällt.

Die bei diesen Fällungsreaktionen erhaltenen Niederschläge sind im allgemeinen chemisch uneinheitlich und enthalten in der Regel Mischungen der Oxide, Oxidhydrate, Hydroxide, Carbonate und/oder Hydrogencarbonate der eingesetzten Metalle.

Die Fällung kann z.B. bei Temperaturen von 20 - 100 °C, besonders 50 - 90 °C, insbesondere bei 60 - 80 °C, durchgeführt werden.

Alternativ kann auch die Metallsalzlösung und die Lauge gleichzeitig in einen Kessel geleitet werden, der die Zirkoniumdioxid-Trägersuspension enthält. Der Träger kann auch in der Metallsalzlösung suspendiert werden und diese dann gleichzeitig mit der Lauge in einen Fällkessel geleitet werden.

Die weitere Katalysatorherstellung wird dann nach bekannten Methoden, also etwa Filtration, Waschung, Trocknung, Calcinierung, Formgebung, Reduktion/Passivierung durchgeführt.

Die auf diese Weise hergestellten Katalysatoren enthalten vor der Reduktion/Passivierung die katalytisch aktiven Metalle in Form eines Gemisches ihrer sauerstoffhaitigen Verbindungen, d.h. insbesondere als Oxide und Mischoxide.

Die hergestellten Katalysatoren können als solche gelagert werden. Vor ihrem Einsatz als Katalysatoren zur Hydrierung von aliphatisch ungesättigten Verbindungen werden sie üblicherweise durch Behandlung mit Wasserstoff vorreduziert. Sie können jedoch auch ohne Vorreduktion eingesetzt werden, wobei sie dann unter den Bedingungen der Dimerisierungsreaktion durch den im Reaktor vorhandenen Wasserstoff reduziert werden. Zur Vorreduktion werden die Katalysatoren im allgemeinen zunächst bei 150 bis 200 °C über einen Zeitraum von 12 bis 20 Stunden einer Stickstoff-Wasserstoff-Atmosphäre ausgesetzt und anschließend noch bis zu ca. 24 Stunden bei 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Das erfindungsgemäße Verfahren wird bevorzugt eingesetzt zur Herstellung eines symmetrischen sekundären Amins der Formel I 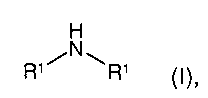 in der
- R¹: Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₂₋₂₀-Hydroxyalkylaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl,
oder R³R⁴N-A-, mit A = C₁₋₆-Alkylen oder -CH₂-CH₂-O-(CH₂-CH₂-O)ₙ-CH₂-CH₂- (mit n = 0, 1 oder 2) und R³, R⁴ = C₁₋₄-Alkyl oder gemeinsam mit dem N-Atom, an das sie gebunden sind, einen Piperidin- oder Morpholinring bildend,
bedeutet,
oder die beiden Reste R¹ gemeinsam -(CH₂)ₗ-CH₂-X-(CH₂)ₘ-, mit
- X: CH₂, CHR⁵, Sauerstoff (O), Schwefel (S) oder NR⁵,
- R⁵: Wasserstoff (H), Alkyl, wie C₁₋₄-Alkyl, Alkylphenyl, wie C₇₋₄₀-Alkylphenyl,
- l, m: eine ganze Zahl von 1 bis 4,
bedeuten, durch Umsetzung eines entsprechenden primären Amins der Formel II oder IIa

R¹―NH₂ (II)

H₂N-(CH₂)ᵢ-CH₂-X-(CH₂)ₘ-NH₂ (IIa).

Die Substituenten R¹, R³ bis R⁵, die Variable X und die Indizes I, m in den Verbindungen I, II und IIa haben unabhängig voneinander folgende Bedeutungen:
R¹:
   - Alkyl, wie C₁₋₂₀₀-Alkyl, bevorzugt C₁₋₂₀-Alkyl, besonders bevorzugt C₁₋₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, isoHexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, insbesondere C₁₋₄-Alkyl,
   - Cycloalkyl, wie C₃₋₁₂-Cycloalkyl, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,
   - Hydroxyalkyl, wie C₁₋₂₀-Hydroxyalkyl, bevorzugt C₁₋₈-Hydroxyalkyl, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)ethyl,
   - Aminoalkyl, wie C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,
   - Hydroxyalkylaminoalkyl, wie C₂₋₂₀-Hydroxyalkylaminoalkyl, bevorzugt C₃₋₈-Hydroxyalkylaminoalkyl, wie (2-Hydroxyethylamino)methyl, 2-(2-Hydroxyethylamino)ethyl und 3-(2-Hydroxyethylamino)propyl,
   - Alkoxyalkyl, wie C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt C₂₋₄-Alkoxyalkyl,
   - Dialkylaminoalkyl, wie C₃₋₃₀-Dialkylaminoalkyl, bevorzugt C₃₋₂₀-Dialkylaminoalkyl, besonders bevorzugt C₃₋₁₀-N,N-Dialkylaminoalkyl, wie (N,N-Dimethylamino)methyl, (N,N-Dibutyl-amino)methyl, 2-(N,N-Dimethylamino)ethyl, 2-(N,N-Diethylamino)ethyl, 2-(N,N-Dibutylamino)ethyl, 2-(N,N-Di-npropylamino)ethyl, 2-(N,N-Di-iso-propylamino)ethyl, (R⁵)₂N-(CH₂)ₗ, ganz besonders 3-(N,N-Dimethylamino)propyl
   - Alkylaminoalkyl, wie C₂₋₃₀-Alkylaminoalkyl, bevorzugt C₂₋₂₀-Alkylaminoalkyl, besonders bevorzugt C₂₋₈-Alkylaminoalkyl, wie Methylaminomethyl, 2-(Methylamino)ethyl, Ethylaminomethyl, 2-(Ethylamino)ethyl und 2-(iso-Propylamino)ethyl, (R⁵)HN-(CH₂)_{q},
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,
   - Aralkyl, wie C₇₋₂₀-Aralkyl, bevorzugt C₇₋₁₂-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
   - Heteroarylalkyl, wie C₄₋₂₀-Heteroarylalkyl, wie Pyrid-2-yl-methyl, Furan-2-ylmethyl, Pyrrol-3-yl-methyl und Imidazol-2-yl-methyl,
   - Alkylaryl, wie C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - Alkylheteroaryl, wie C₄₋₂₀-Alkylheteroaryl, wie 2-Methyl-3-pyridinyl, 4,5-Dimethyl-imidazol-2-yl, 3-Methyl-2-furanyl und 5-Methyl-2-pyrazinyl,
   - R³R⁴N-A-, mit A = C₁₋₆-Alkylen (wie -CH₂-CH₂-, -CH₂-CH₂-CH₂-, -CH₂-CH₂-CH₂-CH₂-) oder -CH₂-CH₂-O-(CH₂-CH₂-O)ₙ-CH₂-CH₂- (mit n = 0, 1 oder 2) und R³, R⁴ = C₁₋₄-Alkyl (wie oben für R¹ definiert) oder gemeinsam mit dem N-Atom, an das sie gebunden sind, einen Piperidin- oder Morpholinring bildend,
   - oder beide Reste bedeuten gemeinsam eine -(CH₂)ₗ-CH₂-X-(CH₂)ₘ- Gruppe, wie -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-,
R⁵:
   - Wasserstoff (H),
   - Alkyl, besonders C₁₋₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
   - Alkylphenyl, besonders C₇₋₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,
X:
   - CH₂, CHR⁵, Sauerstoff (O), Schwefel (S) oder NR⁵, bevorzugt CH₂, NH und O,
l:
   - eine ganze Zahl von 1 bis 4 (1, 2, 3 oder 4), bevorzugt 1 und 2,
m:
   - eine ganze Zahl von 1 bis 4 (1, 2, 3 oder 4), bevorzugt 1 und 2,

Die im erfindungsgemäßen Verfahren einsetzbaren primären Amine können geradkettig, verzweigt oder zyklisch sein. Hinsichtlich der Kohlenstoffzahl der primären Amine gibt es praktisch keine Beschränkungen. Die primären Amine können ferner Substituenten tragen oder funktionelle Gruppen enthalten, welche sich unter den Bedingungen der Dimerisierung zum symmetrischen sekundären Amin unter NH₃-Bildung inert verhalten, beispielsweise Hydroxy-, Alkoxy-, Alkylamino- oder Dialkylaminogruppen, oder gegebenenfalls auch unter den Bedingungen mithydriert werden, beispielsweise aliphatische (d.h. nicht-aromatische) CC-Doppelbindungen und CC-Dreifachbindungen.

Bevorzugt werden im erfindungsgemäßen Verfahren beispielsweise die folgenden primären Amine eingesetzt:
3-(Dimethylamino)propylamin (DMAPA), (Umsetzung zu Bis-[(3-Dimethylamino)propyl]amin (Bis-DMAPA),
1,3-Propandiamin (Umsetzung zu entsprechendem Oligomer oder Polymer).

Das erfindungsgemäße Verfahren wird im allgemeinen bei Temperaturen von 50 bis 250°C, vorzugsweise bei 90 bis 170 °C, besonders bevorzugt bei 120 bis 160°C, und Drücken von 5 bis 350 bar, vorzugsweise 5 bis 200 bar, besonders bevorzugt 10 bis 100 bar, insbesondere 10 bis 30 bar, diskontinuierlich oder bevorzugt kontinuierlich in Druckapparaturen wie Autoklaven oder bevorzugt Rohrreaktoren durchgeführt. Der Druck ist dabei vorzugsweise der Wasserstoffdruck im Reaktor. Bei Verwendung eines Rohrreaktors kann der verwendete Katalysator auch als Festbettkatalysator vorliegen.

Die Umsetzung kann in der Gasphase oder in der Gas-/Flüssigphase erfolgen.

Die Katalysatorbelastung, bezogen auf das eingesetzte primäre Amin, beträgt vorzugsweise 0,1 bis 2 kg l⁻¹h⁻¹, insbesondere 0,8 bis 1,2 kg l⁻¹h⁻¹. Es kann ein Teil des flüssigen oder gasförmigen Reaktionsaustrags in die Umsetzung zurückgeführt werden.

Das erfindungsgemäße Verfahren lässt sich lösungsmittelfrei oder in Lösungsmitteln wie z.B. Wasser, Methanol, Ethanol, Tetrahydrofuran (THF), Methyl-tert.-butylether (MTBE) oder N-Methylpyrrolidon (NMP) durchführen. Im Lösungsmittel kann dabei das eingesetzte primäre Amin gelöst sein. Das Lösungsmittel kann auch getrennt dem Reaktor an beliebiger Stelle zugeführt werden. Vorzugsweise wird lösungsmittelfrei gearbeitet.

Das mit dem erfindungsgemäßen Verfahren erhaltene gewünschte symmetrische sekundäre Amin lässt sich in an sich bekannter Weise, beispielsweise destillativ, vom Reaktionsgemisch abtrennen und reinigen.

Beispielsweise ist es bei der Aufarbeitung des Reaktionsprodukts möglich, durch Rektifikation einen Strom mit reinem sekundärem Amin und einen Strom mit primärem Amin zu erhalten, wobei der Strom mit dem primären Amin vorteilhafterweise in die Synthese zurückgeführt wird.

Erfindungsgemäß fallen im allgemeinen im Reaktionsrohprodukt die (unumgesetzten) primären Amine und die symmetrischen sekundären Amine in einem Gewichtsverhältnis von 10:1 bis 1:10, vorzugsweise 2 : 3-4, an.

Das erfindungsgemäße Verfahren gestattet es vorteilhafterweise, Reaktionsrohprodukte zu erhalten, die nur geringe Mengen an tertiären Aminen als Reaktionsprodukte enthalten, in der Regel in Mengen < 10 Gew.-%, insbesondere < 5 Gew.-%, ganz besonders 0 bis 3 Gew.-%.

Die mit dem erfindungsgemäßen Verfahren erhältlichen symmetrischen sekundären Amine, wie z.B. Bis-DMAPA, können eingesetzt werden als Härter für Epoxyharze, Katalysatoren für Polyurethane, Zwischenprodukte zur Herstellung quaternärer Ammoniumverbindungen, Weichmacher, Korrosionsinhibitoren, Textilhilfsmittel, Farbstoffe und/oder Emulgatoren. Die symmetrischen sekundären Amine können außerdem zur Herstellung von Kunstharzen, Ionenaustauschern, Pharmazeutika, Pflanzenschutzund/oder Schädlingsbekämpfungsmitteln dienen.

### Beispiele

### Herstellung von Katalysatoren

### Katalysator A (erfindungsgemäß):

Katalysator A wurde durch Auffällung der Komponenten Cu, Co, Ni auf monoklines Zirkoniumdioxid, das im Fällkessel vorgelegt wurde, wie folgt hergestellt:

In einem Rührkessel aus Glas wurde eine Suspension aus 155 g monoklinem Zirkoniumdioxid-Pulver (BET = 105 m² g⁻¹) in 2 l Wasser vorgelegt und unter Rühren auf 70 °C erhitzt. Innerhalb von 30 Min. wurde dann eine Lösung aus 190,1 g Cu(NO₃)₂ x 2,5 H₂O, 561,9 g Ni(NO₃)₂ x 6 H₂O und 543,7 g Co(NO₃)₂ x 6 H₂O in 2,8 l Wasser zugetropft. Durch gleichzeitiges Zutropfen einer 20 %-igen Sodalösung (700 g Na₂CO₃ in 2,8 l Wasser) wurde der pH bei konstant 6,0 gehalten. Nach der Zugabe der Lösungen wurde für 1 h bei 70 °C nachgerührt und schließlich durch Zugabe von Sodalösung der pH-Wert auf 7,4 erhöht. Die Suspension wurde auf eine Nutsche gepumpt und mit 100 l Wasser gewaschen. Der Filterkuchen wurde für 16 h bei 200 °C im Trockenschrank getrocknet, anschließend auf Korngrößen < 1,6 mm zerkleinert und für 2 h bei 400°C in einem Drehrohrofen im Luftstrom (150 l/h) calciniert.

Das so erhaltene Katalysatorpulver hatte die Zusammensetzung:
28 Gew.-% Ni, berechnet als NiO,
28 Gew.-% Co, berechnet als CoO,
11 Gew.-% Cu, berechnet als CuO, und
33 Gew.-% Zr, berechnet als ZrO₂.

Das Pulver wurde mit 3 Gew.-% Graphit versetzt, kompaktiert, wieder auf < 1,6 mm zerkleinert, und schließlich zu 4,75 x 3 mm-Tabletten gepresst. Die Tabletten wurden für 2 h bei 400°C im Muffelofen an Luft nachcalciniert. In einer Reduktionskolonne wurden die Tabletten dann in einem Wasserstoff/Stickstoffstrom reduziert, zunächst 6 h bei 150°C, dann 6 h bei 280°C. Nach Abkühlung auf Raumtemperatur wurden die Tabletten dann in einem verdünnten Luftstrom passiviert.

### Katalysator B (Vergleichskatalysator):

Der Vergleichskatalysator wurde wie der erfindungsgemäße Katalysator A hergestellt, mit dem Unterschied, dass kein monoklines Zirkoniumdioxid vorgelegt wurde. Anstelle dessen wurden zu der Cu-, Co- und Ni-Nitrathaltigen Metallsalzlösung 775 g einer Zirkoniumacetatlösung, enthaltend 20 Gew.-% ber. ZrO₂, (bezogen auf das Gewicht der Zirkoniumacetatlösung) zugegeben (Mischfällung). Die weitere Herstellung erfolgte analog Katalysator A. Das analog erhaltene Katalysatorpulver hatte die gleiche Zusammensetzung wie beim Katalysator A beschrieben.

### Beispiel 1:

Zur Testung der mechanischen Stabilität der Katalysatoren A und B unter den Reaktionsbedingungen der hydrierenden Aminierung von hydroformylierten Polyisobuten (PIB-Oxo; Molmasse: 1000) zum primären Amin PIBA nach dem Reaktionsschema 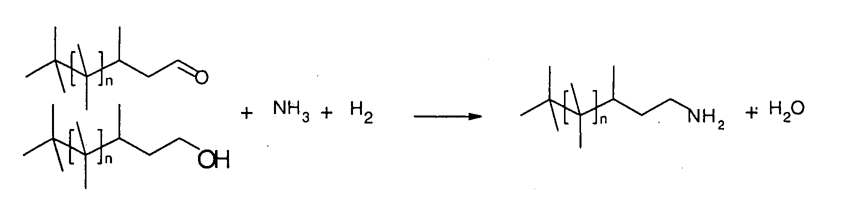 wurden die Katalysatoren in einem Autoklaven einem sog. Kochtest (Autoklaventest) unterzogen, bei dem die Reaktionsbedingungen wie folgt nachgestellt wurden:
20 g Katalysator wurden in einen Drahtkorb in einem Rührautoklaven gegeben. Dazu wurden 150 ml einer PIBA/Mihagol (50/50) Lösung gegeben, so dass die Katalysatortabletten gut mit Flüssigkeit überdeckt waren. Der Autoklav wurde verschlossen, mit Stickstoff gespült, der Rührer mit 700 U/min betrieben, 50 bar H₂ aufgepresst und auf 200 °C aufgeheizt. Der Druck wurde dann durch Zupressen von H₂ auf 200 bar eingestellt. Unter diesen Bedingungen wurde der Katalysator für unterschiedliche Zeiträume behandelt. Anschließend wurde abgekühlt, vorsichtig entspannt und die mechanische Stabilität des Katalysators durch Messung der Seitendruckfestigkeit (SDF) bestimmt.

Hierzu wurde die Katalysatortablette zwischen zwei parallelen Platten auf der Mantelseite mit zunehmender Kraft belastet, bis Bruch eintrat. Die beim Bruch registrierte Kraft ist die Seitendruckfestigkeit. Die Bestimmung erfolgte auf einem Prüfgerät der Firma Zwick, Ulm, mit festsitzendem Drehteller und frei beweglichem, vertikalem Stempel, der den Formkörper gegen den festsitzenden Drehteller drückte. Der frei bewegliche Stempel war mit einer Druckmessdose zur Aufnahme der Kraft verbunden. Das Gerät wurde durch einen Computer gesteuert, welcher die Meßwerte registrierte und auswertete. Aus einer gut durchmischten Katalysatorprobe wurden 25 einwandfreie (d.h. rissefrei und ohne abgestoßenen Kanten) Tabletten entnommen, deren Seitendruckfestigkeit ermittelt und anschließend gemittelt.

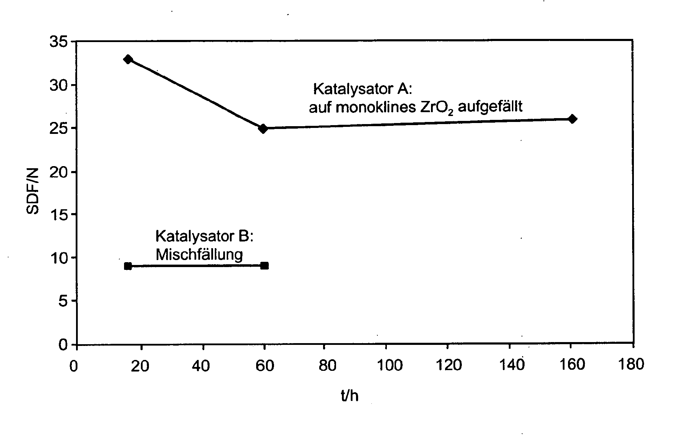

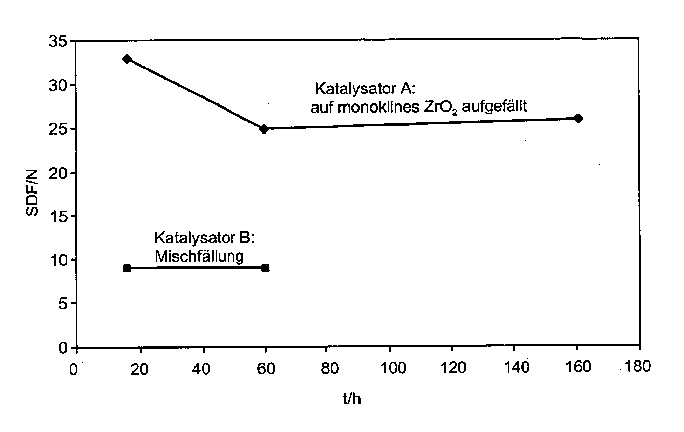

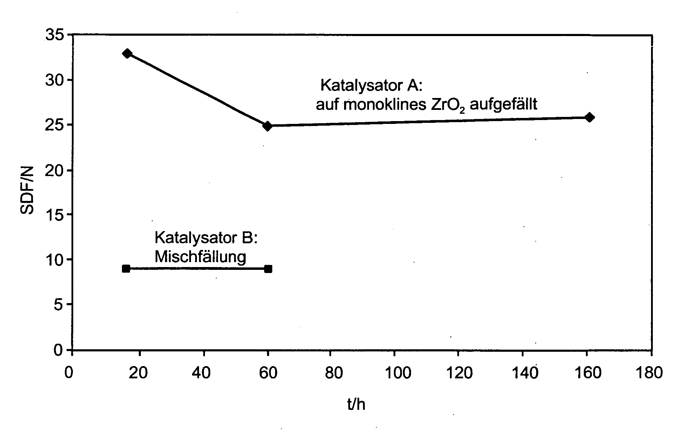

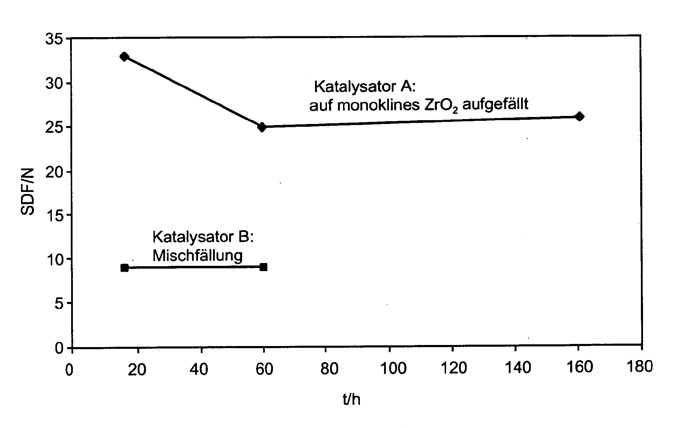

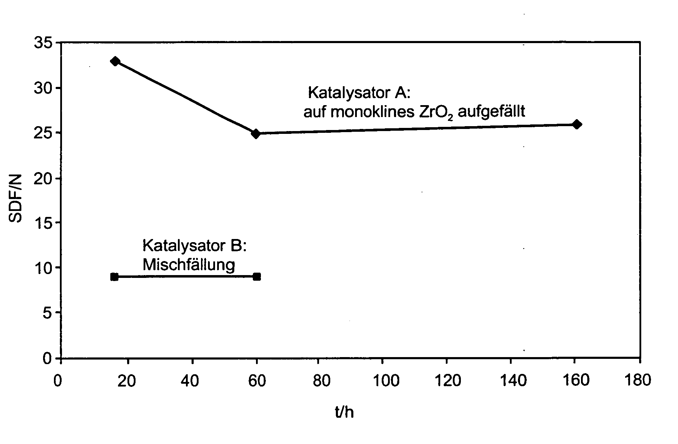

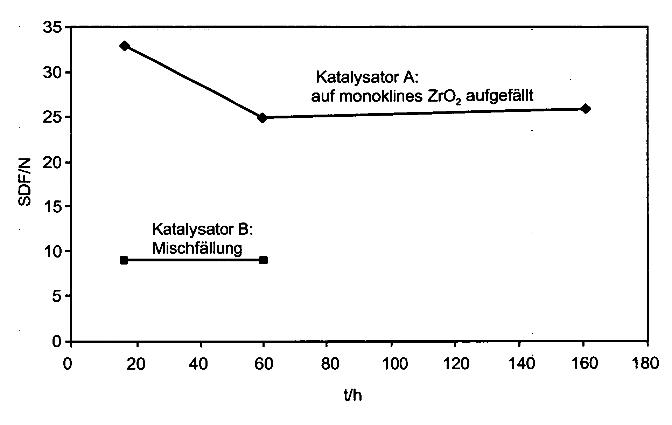

In der beiliegenden Abbildung 1 sind die Seitendruckfestigkeiten (Einheit: Newton, N) der beiden Katalysatoren A und B über die Dauer des Autoklaventests (in Stunden) aufgetragen.

Bei dem durch Mischfällung hergestellten Kontakt B zeigte das Röntgendiffraktogramm nach erfolgtem Kochtest, dass sich die zunächst röntgenamorphe ZrO₂-Phase in tetragonales und monoklines ZrO₂ umgewandelt hatte. Bei Katalysator A wurde keine Umkristallisation (= Änderung der Modifikation) festgestellt.

### Beispiel 2:

Ein beheizter Rohrreaktor mit 10 mm Innendurchmesser, einem zentral angebrachten Thermoelement und einer Gesamtlänge von 35 cm wird mit 90 g des Katalysators A gefüllt.

Vor der Reaktion wird der Katalysator bei ca. 180°C aktiviert, zunächst in einem Gasstrom von Stickstoff und Wasserstoff im Volumenverhältnis 4:1, anschließend in einem Gasstrom von Stickstoff und Wasserstoff im Volumenverhältnis 1:1 und schließlich in reinem Wasserstoff.

Durch den Reaktor werden von unten nach oben 1200 g/(l*h) an 3-(Dimethylamino)propylamin (DMAPA) und 20 Nl/h (Nl = Normliter = auf Normalbedingungen umgerechnetes Volumen) Wasserstoff zugegeben. Der Reaktor wird bei einer Temperatur von 140 °C und einem Gesamtdruck von 30 bar gehalten.

Das aus dem Reaktor austretende Gemisch wird abgekühlt und auf Normaldruck entspannt. Der Reaktoraustrag enthält als Hauptkomponente das gewünschte Bis-[(3-Dimethylamino)propyl]amin (Bis-DMAPA), neben unumgesetzten 3-(Dimethylamino)propylamin (DMAPA) und geringen Mengen verschiedener Nebenprodukte.

In einer Labordestillationsapparatur mit Füllkörperschüttung (10 theoretische Trennstufen) wird anschließend der flüssige Reaktoraustrag unter vermindertem Druck und bei einem Rücklaufverhältnis von ca. 5:1 diskontinuierlich destilliert. Es wird eine Fraktion mit einem Gehalt von > 97 Gew.-% an DMAPA und eine zweite Fraktion mit einem Gehalt von > 98 Gew.-% an Bis-DMAPA erhalten.

## Patentansprüche

1. Verfahren zur Herstellung eines symmetrischen sekundären Amins durch Umsetzung eines primären Amins in Gegenwart von Wasserstoff und eines Katalysators, **dadurch gekennzeichnet, dass** man einen Katalysator einsetzt, bei dessen Herstellung eine Fällung von katalytisch aktiven Komponenten auf monoklines, tetragonales oder kubisches Zirkoniumdioxid erfolgte.

2. Verfahren nach dem vorhergehenden Anspruch, **dadurch gekennzeichnet, dass** es sich bei den aufgefällten katalytisch aktiven Komponenten um Metallsalze handelt, wobei das Metall aus den Nebengruppen VIII und IB des Periodensystems ausgewählt ist.

3. Verfahren nach dem vorhergehenden Anspruch, **dadurch gekennzeichnet, dass** es sich bei den Metallsalzen um in Wasser schwer- oder unlösliche, basische Salze handelt.

4. Verfahren nach einem der beiden vorhergehenden Ansprüche, **dadurch gekennzeichnet, dass** es sich bei den Salzen um Oxide, Oxidhydrate, Hydroxide, Carbonate und/oder Hydrogencarbonate handelt.

5. Verfahren nach einem der Ansprüche 2 bis 4, **dadurch gekennzeichnet, dass** das Metall aus der Gruppe Fe, Co, Ni, Ru, Rh, Pd, Pt und Cu ausgewählt ist.

6. Verfahren nach einem der Ansprüche 2 bis 4, **dadurch gekennzeichnet, dass** das Metall aus der Gruppe Cu, Ni und Co ausgewählt ist.

7. Verfahren nach einem der vorhergehenden Ansprüche, **dadurch gekennzeichnet, dass** die katalytisch aktive Masse des Katalysators vor der Behandlung mit Wasserstoff
20 bis 85 Gew.-% sauerstoffhaltige Verbindungen des Zirkoniums, berechnet als ZrO₂,
1 bis 30 Gew.-% sauerstoffhaltige Verbindungen des Kupfers, berechnet als CuO, und
14 bis 70 Gew.-% sauerstoffhaltige Verbindungen des Nickels, berechnet als NiO, enthält.

8. Verfahren nach einem der vorhergehenden Ansprüche, **dadurch gekennzeichnet, dass** die katalytisch aktive Masse des Katalysators vor der Behandlung mit Wasserstoff
20 bis 65 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, und
15 bis 50 Gew.-% sauerstoffhaltige Verbindungen des Kobalts, berechnet als CoO, enthält.

9. Verfahren nach einem der Ansprüche 5 bis 8, **dadurch gekennzeichnet, dass** das Molverhältnis von Nickel zu Kupfer größer 1 ist.

10. Verfahren nach einem der vorhergehenden Ansprüche, **dadurch gekennzeichnet, dass** das monokline, tetragonale oder kubische Zirkoniumdioxid ein oder mehrere Metalloxide der Nebengruppe IIIB oder der Hauptgruppe IIA des Periodensystems enthält.

11. Verfahren nach einem der vorhergehenden Ansprüche, **dadurch gekennzeichnet, dass** man die Umsetzung bei Temperaturen von 50 bis 250 °C durchführt.

12. Verfahren nach einem der vorhergehenden Ansprüche, **dadurch gekennzeichnet, dass** man die Umsetzung bei Drücken von 5 bis 350 bar in der Gas/Flüssigphase oder in der Gasphase durchführt.

13. Verfahren nach einem der vorhergehenden Ansprüche zur Herstellung eines symmetrischen sekundären Amins der Formel I 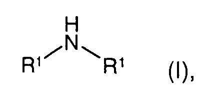 in der
R¹ Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₂₋₂₀-Hydroxyalkylaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl,
oder R³R⁴N-A-, mit A = C₁₋₆-Alkylen oder -CH₂-CH₂-O-(CH₂-CH₂-O)ₙ-CH₂-CH₂- (mit n = 0, 1 oder 2) und R³, R⁴ = C₁₋₄-Alkyl oder gemeinsam mit dem N-Atom, an das sie gebunden sind, einen Piperidin- oder Morpholinring bildend,
bedeutet,
oder die beiden Reste R¹ gemeinsam -(CH₂)ₗ-CH₂-X-(CH₂)ₘ-, mit
X CH₂, CHR⁵, Sauerstoff (O), Schwefel (S) oder NR⁵,
R⁵ Wasserstoff (H), Alkyl, wie C₁₋₄-Alkyl, Alkylphenyl, wie C₇₋ ₄₀-Alkylphenyl,
l, m eine ganze Zahl von 1 bis 4,
bedeuten, durch Umsetzung eines entsprechenden primären Amins der Formel II oder IIa
R¹―NH₂ (II)
H₂N-(CH₂)ₗ-CH₂-X-(CH₂)ₘ-NH₂ (IIa)

14. Verfahren nach einem der vorhergehenden Ansprüche 1 bis 12 zur Herstellung eines symmetrischen sekundären Amins der Formel I 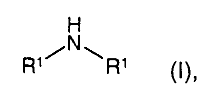 in der
R¹ C₃₋₁₀-Dialkylaminoalkyl, wie 3-(N,N-Dimethylamino)propyl,
bedeutet, durch Umsetzung eines entsprechenden primären Amins der Formel II
R¹―NH₂ (II).

15. Verwendung eines Katalysators, definiert wie in einem der Ansprüche 1 bis 10, zur Herstellung eines symmetrischen sekundären Amins durch Umsetzung eines primären Amins in Gegenwart von Wasserstoff.
